# EUROPEAN PATENT APPLICATION

(11) **EP 4 782 023 A1**
(43) Date of publication of application: **29.07.2026**
(21) Application number: 26153143.8
(22) Date of filing: 21.01.2026
(51) Int. Cl.: A61M 1/16

(54) **MODULAR OXYGENATOR SYSTEM**

(30) Priority: 22.01.2025 US 202563748223 P
(71) Applicant: Abiomed, Inc., Danvers, MA 01923 (US)
(72) Inventor: LUDDY, Charles, 01923 Danvers (US)
(74) Representative: Wittmer, Maximilian

(57) **Abstract**

A modular blood-gas exchange system is provided. The system may include a first oxygenator manifold, a second oxygenator manifold, and a plurality of blood-gas exchange cartridges removably coupled to the first and second oxygenator manifolds. The first oxygenator manifold may include a plurality of first blood ports. The second oxygenator manifold may include a plurality of second blood ports. Each blood-gas exchange cartridges may be configured to allow blood and a sweep gas to flow through the removably attachable cartridge, and may be configured to allow a gas molecule to transfer between the sweep gas and the blood.

## Description

### CROSS-REFERENCE TO RELATED APPLICATIONS

The present application claims priority to U.S. Provisional Patent Application No. 63/748,223, filed January 22, 2025, the contents of which are incorporated herein in its entirety.

### TECHNICAL FIELD

The present disclosure is drawn to the field of blood-gas exchange, and specifically oxygenator systems utilizing cartridges.

### BACKGROUND

Various therapies rely on oxygenators, which allow the extracorporeal oxygenation of blood, and the removal of carbon dioxide from blood. When a conventional oxygenator needs to be exchanged and replaced, the therapy may need to be halted. In this regard, the oxygenator may be cut out of the circuit, a new oxygenator may be plumbed into the circuit, and therapy is then resumed.

### BRIEF SUMMARY

The disclosed system, kit, and method may eliminate the interruption in therapy and significantly simplifies the exchange process. Further, the disclosed system, kit, and method may provide for more flexibility in choices with regards to how much oxygenation can be provided, by allowing the addition or removal of oxygenator capacity based on patient demand.

In some embodiments, a system for blood-gas exchange is provided. The system may include a first oxygenator manifold, a second oxygenator manifold, and a plurality of blood-gas exchange cartridges removably coupled to the two oxygenator manifolds. The first oxygenator manifold may include a plurality of first blood ports, and the second oxygenator manifold may include a plurality of second blood ports. Each blood-gas exchange cartridges may be configured to allow blood and a sweep gas to flow through the removably attachable cartridge and allow a gas molecule to transfer between the sweep gas and the blood.

The first oxygenator manifold may include a blood inlet where each of the first blood ports may be operably connected to the blood inlet. The first oxygenator manifold may include at least one valve (such as a plurality of valves) where each of the first blood ports is operably connected to the at least one valve (or at least one valve of a plurality of valves).

The second oxygenator manifold may include a blood outlet where each of the first blood ports may be operably connected to the blood outlet. The second oxygenator manifold may include at least one valve (such as a plurality of valves) where each of the first blood ports is operably connected to the at least one valve (or at least one valve of a plurality of valves).

Each oxygenator manifold may be capable of removably connecting to 5 or more blood-gas exchange cartridges. The system may be configured to operate simultaneously using at least two, but less than all, of the plurality of blood-gas exchange cartridges.

Each blood-gas exchange cartridges may be configured to be removably coupled to at least one of the plurality of first blood ports and at least one of the plurality of second blood ports. Each blood-gas exchange cartridge may include at least one sensor. Each blood-gas exchange cartridge may be configured to have a gas exchange surface area of between 1.5 m² and 2.5 m². Each blood-gas exchange cartridge may be configured to have a gas exchange surface area of at between 0.5 m² and 1.5 m².

The plurality of blood-gas exchange cartridges may include a first blood-gas exchange cartridge and a second blood-gas exchange cartridge, wherein the first and second blood-gas exchange cartridges are arranged in parallel. The first oxygenator manifold and the second oxygenator manifold may be expandable to allow a third blood-gas exchange cartridge to be added to the system, the third blood-gas exchange cartridge being operably coupled to the first oxygenator manifold and the second oxygenator manifold. Each blood-gas exchange cartridge may be disposable.

The system may include a number C of blood-gas exchange cartridges, and a number V of valves, such that C > V. The system may include a number C of blood-gas exchange cartridges, and a number V of valves, such that C = V. The system may include a number C of blood-gas exchange cartridges, and a number V of valves, such that C < V.

The system may be configured such that blood flows through each of a first and second blood-gas exchange cartridge simultaneously. The system may be configured such that blood flows through a first or a second blood-gas exchange cartridge while the other of the first or second blood-gas exchange cartridge is configured to remain in standby mode.

The system may include a controller configured to control at least one valve of the system. The controller may be configured to control at least one valve of the system in response to information received from the at least one sensor.

The system may include a first connector operably connected to a blood inlet and a second connector operably connected to a blood outlet. The first connector may be removably coupled to a blood-gas exchange cartridge, the first oxygenator manifold, or both. The second connector may be removably coupled to a blood-gas exchange cartridge, the second oxygenator manifold, or both. and is configured to be removably coupled from the second oxygenator manifold. The first connector and the second connector may be configured to form a fluid tight seal. The first and second connectors may each include single use connectors. The first connector and the second connector may each be configured to lock into place when connected.

The system may include tubing operably coupled to the plurality of blood-gas exchange cartridges. The system may include a heat exchanger operably coupled to the plurality of blood-gas exchange cartridges. The system may include a pump operably coupled to the plurality of blood-gas exchange cartridges.

In some embodiments, a kit is provided. The kit may include a first oxygenator manifold that includes a plurality of first blood ports, a second oxygenator manifold that includes a plurality of second blood ports, and a plurality of blood-gas exchange cartridges. Each blood-gas exchange cartridge may be configured to be removably coupled to the first and second oxygenator manifolds, allow blood and a sweep gas to flow through the removably attachable cartridge, and allow a gas molecule to transfer between the sweep gas and the blood.

The kit may include tubing configured to be operably coupled to the plurality of blood-gas exchange cartridges. The kit may include a heat exchanger. The kit may include a pump.

The first oxygenator manifold may include a blood inlet where each of the first blood ports may be operably connected to the blood inlet. The first oxygenator manifold may include at least one valve (such as a plurality of valves) where each of the first blood ports is operably connected to the at least one valve (or at least one valve of a plurality of valves).

The second oxygenator manifold may include a blood outlet where each of the first blood ports may be operably connected to the blood outlet. The second oxygenator manifold may include at least one valve (such as a plurality of valves) where each of the first blood ports is operably connected to the at least one valve (or at least one valve of a plurality of valves).

Each oxygenator manifold may be capable of removably connecting to 5 or more blood-gas exchange cartridges.

Each blood-gas exchange cartridge may be configured to be removably coupled to at least one first blood port and at least one second blood port. Each blood-gas exchange cartridge may include at least one sensor. Each blood-gas exchange cartridge may be configured to have a gas exchange surface area of between 1.5 m² and 2.5 m². Each blood-gas exchange cartridge may be configured to have a gas exchange surface area of at between 0.5 m² and 1.5 m². Each blood-gas exchanger may be disposable.

The kit may include a number C of blood-gas exchange cartridges, and a number V of valves, such that C > V. The kit may include a number C of blood-gas exchange cartridges, and a number V of valves, such that C = V. The kit may include a number C of blood-gas exchange cartridges, and a number V of valves, such that C < V.

The kit may include a controller configured to control at least one valve. The controller may be configured to control at least one valve in response to information received from at least one sensor.

The kit may include a first connector operably connected to a blood inlet and a second connector operably connected to a blood outlet. The first connector may be removably coupled to a blood-gas exchange cartridge, the first oxygenator manifold, or both. The second connector may be removably coupled to a blood-gas exchange cartridge, the second oxygenator manifold, or both. and is configured to be removably coupled from the second oxygenator manifold. The first connector and the second connector may be configured to form a fluid tight seal. The first and second connectors may each include single use connectors. The first connector and the second connector may each be configured to lock into place when connected. The first connector, the second connector, or both may include a single-use connection. The first connector, the second connector, or both may include a reusable connection.

In some embodiments, a method is provided. The method may include flowing a gas through a first blood-gas exchange cartridge of a plurality of blood-gas exchange cartridges, each blood gas cartridge being removable connected to first and second oxygenator manifolds. The method may include, while the gas is flowing through the first blood-gas exchange cartridge, flowing blood through the first oxygenator manifold. The first oxygenator manifold may include a plurality of first blood ports, at least one first blood port removably connected to the first blood-gas exchange cartridge, the first blood-gas exchange cartridge further removably connected to a blood port of a plurality of second blood ports of the second oxygenator manifold.

The method may include flowing blood through a second blood-gas exchange cartridge of the plurality of blood gas exchange cartridges. The method may include stopping blood flow through the first blood gas-exchange cartridge after flowing blood through the second blood-gas exchange cartridge. The method may include removing the first blood-gas exchange cartridge from the first and second oxygenator manifolds, e.g., while blood is flowing through the second blood-gas exchange cartridge.

The method may include connecting a third blood-gas exchange cartridge to the first and second oxygenator manifolds. The method may include flowing blood through the third blood gas-exchange cartridge while blood is flowing through the first blood-gas exchange cartridge.

The method may include receiving information from a first sensor, where the first sensor is configured to determine a status of a blood-gas exchange cartridge. The method may include determining if a blood-gas exchange cartridge is attached based on the information from the first sensor. The method may include determining if an unused blood-gas exchange cartridge is attached based on the information from the first sensor. The method may include generating an alarm responsive to a determination a blood-gas exchange cartridge is not attached, a determination an unused blood-gas exchange cartridge is not attached, or both. The method may include controlling a valve based on the information received from the first sensor.

### BRIEF DESCRIPTION OF DRAWINGS

The accompanying drawings, which are incorporated in and constitute a part of this specification, illustrate embodiments of the present invention and, together with a general description of the invention given above, and the detailed description of the embodiments given below, serve to explain the principles of the present invention.
Figure 1 is an exploded view of an embodiment of a system.
Figure 2 is an exploded view of a portion of an embodiment of a system, utilizing connectors.
Figure 3 is a schematic illustration of a blood-gas exchange cartridge.
Figure 4 is a schematic illustration of an embodiment of a system.
Figure 5 is an illustration of an embodiment of a system.
Figure 6 is a flowchart of an embodiment of a method.

It should be understood that the appended drawings are not necessarily to scale, presenting a somewhat simplified representation of various features illustrative of the basic principles of the invention. The specific design features of the sequence of operations as disclosed herein, including, for example, specific dimensions, orientations, locations, and shapes of various illustrated components, will be determined in part by the particular intended application and use environment. Certain features of the illustrated embodiments have been enlarged or distorted relative to others to facilitate visualization and clear understanding. In particular, thin features may be thickened, for example, for clarity or illustration.

### DETAILED DESCRIPTION

Interruptions or pauses in therapy may create challenges, especially with high demand patients. The process of cutting out and replacing an oxygenator may be very crude - it may take multiple people to perform the exchange, it is complex, and as it involves blood, may involve the cleanup of potentially infectious materials.

Centers and/or users that are less familiar with oxygenators will sometimes not deploy treatments that utilize oxygenators (e.g., extracorporeal membrane oxygenation (ECMO) treatments) because of the challenges associated with this process.

In some embodiments, a flexible, easy-to-operate, expandable system for blood-gas exchange is provided.

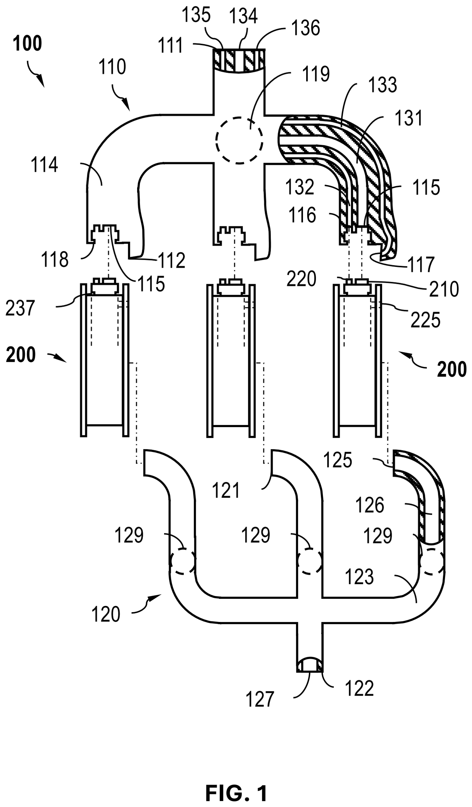

Referring to FIG. 1, a system **100** may include a first oxygenator manifold **110,** a second oxygenator manifold **120,** and a plurality of blood-gas exchange cartridges **200** that are removably coupled to the first and second oxygenator manifolds.

The first oxygenator manifold **110** may include a plurality of first blood ports **115.** In some embodiments, the first oxygenator manifold may be configured such that blood flows through the manifold from a first end **111** towards a second end **112.** In some embodiments, the first oxygenator manifold may include a blood inlet **134** at a first end **111** of the first oxygenator manifold **110.** In some embodiments, each first blood port **115** may be operably connected to the blood inlet **113.**

In some embodiments, the first oxygenator manifold **110** may include one or more arms **114.** In some embodiments, each arm may include at least one lumen **131, 132, 133** extending therethrough.

In some embodiments, each arm in the first oxygenator manifold may include one first blood port **115.** In some embodiments, a lumen **131** operably couples the first blood port **115** of the arm **114** to the blood inlet **134.**

In some embodiments, each arm in the first oxygenator manifold may include a first sweep gas port **116.** In some embodiments, a lumen **132** operably couples the first sweep gas port **116** of the arm **114** to a sweep gas inlet **135,** which is operably connected to a sweep gas source (not shown).

In some embodiments, each arm in the first oxygenator manifold may include a second sweep gas port **117.** In some embodiments, a lumen **133** operably couples the second sweep gas port **117** of the arm **114** to a sweep gas outlet **136.**

As will be understood, the exact arrangement of the sweep gas ports, if used, may vary according to the exact design of the gas cartridge. For example, in some embodiments, the first oxygenator manifold may include the first sweep gas port, and the second oxygenator manifold may include the second sweep gas port. In some embodiments, the first and second gas ports may be included in the second oxygenator manifold.

In some embodiments, the second end **112** of the first oxygenator manifold **110** may be configured to interact with each removably-attached blood gas exchange cartridge **200.** In some embodiments, the second end **112** may include one or more protrusions or depressions **118** configured to interact with one or more protrusions or depressions **237** of the blood-gas exchange cartridge **200.** For example, second end may define an external surface that is configured to slidably receive a blood-gas exchange cartridge where protrusions **118** interact with depressions **237** in order to position the blood-gas exchange cartridge correctly.

The second oxygenator manifold **120** may include a plurality of second blood ports **125.**

In some embodiments, the first oxygenator manifold may be configured such that blood flows through the second oxygenator manifold from a first end **121** towards a second end **122.** In some embodiments, the second oxygenator manifold may include a blood outlet **127** at a second end **122** of the first oxygenator manifold **110.** In some embodiments, each second blood port **125** may be operably connected to the blood inlet **113.**

In some embodiments, the second oxygenator manifold may include a plurality of arms 123. In some embodiments, each arm may include a second blood port **125.** In some embodiments, each arm may define at least one lumen **126** extending therethrough. In some embodiments, a lumen **126** may operably couple a second blood port **125** to a blood outlet **127.**

In some embodiments, each manifold **110, 120** may be configured to control the flow of blood through manifold using one or more valves **119, 129.** In some embodiments, the first oxygenator manifold may include at least one valve **119.** In some embodiments, the first oxygenator manifold may include a plurality of valves. In some embodiments, the second manifold may include at least one valve **129.** In some embodiments, the second manifold may include a plurality of valves. In some embodiments, the first and second oxygenator manifolds each include at least one valve. In some embodiments, the first and second oxygenator manifolds each include a plurality of valves. For example, in some embodiments, a single valve may be used as a "selector" to select which arm the blood is allowed to flow through. In some embodiments, one valve may be positioned in or coupled to each arm of a given manifold.

The system may include a number *C* of blood-gas exchange cartridges, and a number *V* of valves. In some embodiments, *C* > *V*. In some embodiments, *C* = *V*. In some embodiments, *C* < *V*.

In some embodiments, the first oxygenator manifold and the second oxygenator manifold may be configured to hold a maximum number *C* of blood-gas exchange cartridges at any single point in time, and the two manifolds may together utilize a number *V* of valves. In some embodiments, *C* > *V*. In some embodiments, *C* = *V*. In some embodiments, *C* < *V*.

In some embodiments, the plurality of blood-gas exchange cartridges includes a first blood-gas exchange cartridge and a second blood-gas exchange cartridge, where the first and second blood-gas exchange cartridges may be arranged in parallel. In some embodiments, blood may be configured to flow through each of the first and second blood-gas exchange cartridge simultaneously.

In some embodiments, blood may be configured to flow through one of the first and second blood-gas exchange cartridges while the other of the first and second blood-gas exchange cartridges is configured to remain in a "standby" mode.

In some embodiments, blood may be configured to flow through a first blood-gas exchange cartridges, until that cartridge is determined to be at or nearing the end of its expected life, at which time the system may be automatically reconfigured to allow blood to flow through both the first and second blood-gas exchange cartridges, or only though the second blood-gas exchange cartridge.

In some embodiments, the plurality of blood-gas exchange cartridges includes a first blood-gas exchange cartridge and a second blood-gas exchange cartridge, where the first and second blood-gas exchange cartridges may be arranged serially.

In some embodiments, the system may be configured to operate using a single blood-gas exchange cartridge. In some embodiments, the system may be configured to operate simultaneously using at least two, but less than all, of the plurality of blood-gas exchange cartridges. For example, if the system is configured to hold 6 cartridges, the system may be configured to operating using any 2 cartridges, with 2 configured to be on "standby", and the fifth cartridges positioned in case one of the two standby cartridges fails unexpectedly after the standby cartridges are activated but before the first two (now used) cartridges are replaced.

In some embodiments, the first oxygenator manifold and the second oxygenator manifold may be expandable to allow a third blood-gas exchange cartridge to be added to the system. In some embodiments, the third blood-gas exchange cartridge may be added to the system while blood is flowing through the first and/or the second blood-gas exchange cartridge.

In some embodiments, the first and/or second oxygenator manifold may be capable of removably connecting to 3 or more blood-gas exchange cartridges. In some embodiments, the first and/or second oxygenator manifold may be capable of removably connecting to 4 or more blood-gas exchange cartridges. In some embodiments, the first and/or second oxygenator manifold may be capable of removably connecting to 5 or more blood-gas exchange cartridges.

In some embodiments, the first and/or second oxygenator manifold may be capable of removably connecting from 3, 4, or 5 blood-gas exchange cartridges up to 5, 6, 7, 8, 9, or 10 blood-gas exchange cartridges, including all combinations and subranges thereof.

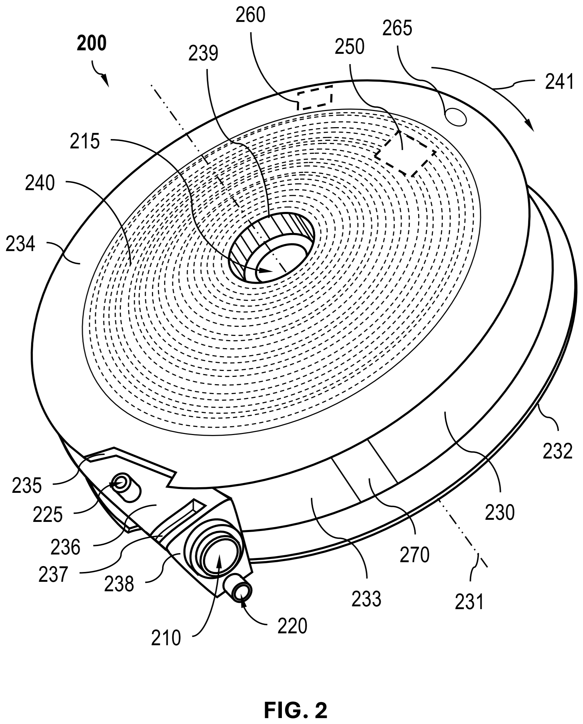

Referring to FIG. 2, each of the plurality of blood-gas exchange cartridges **200** may be configured to allow blood and a sweep gas to flow through the removably attachable cartridge, and may be configured to allow a gas molecule to transfer between the sweep gas and the blood.

In some embodiments, each blood-gas exchange cartridge **200** may be configured to have a housing **230.** The hosing may define a first outer surface 234, and a second outer surface 232, separated by a middle portion **233,** the middle portion. In some embodiments, the first outer surface **234** and the second outer surface **232** may have a larger diameter than the middle portion **233.** In some embodiments, the first and/or second outer surfaces may include one or more notches **235.** In some embodiments, the housing may define an arm **236** extending away from the main body of the cartridge, in a tangential direction. In some embodiments, the housing may define one or more protrusions or depressions **237.** In some embodiments, the one or more protrusions or depressions may be configured to interact with one or more depressions or protrusions on the first or second oxygenator manifold. In some embodiments, the protrusions or depressions **237** may be configured to allow the cartridge to be slidably received by the first or second oxygenator manifold in a direction orthogonal to a central axis **231.**

The housing **230** may define a blood inlet **210** and a blood outlet **215.** In some embodiments, the housing may define an external surface **238** that defines the blood inlet **210.** In some embodiments, the housing may define surface **234** that defines an opening **239** that includes the blood outlet **215.**

In some embodiments, the cartridge may be configured to cause the blood to flow in a first direction **241** (e.g., clockwise) around the central axis **231.** In some embodiments, blood is configured to spiral around the central axis as it flows from the blood inlet **210** to the blood outlet **215.** In some embodiments, the cartridge may be configured such that blood flows from an outer edge of the cartridge towards a centrally-located blood outlet **215.** In some embodiments, the cartridge may be configured such that blood enters the blood inlet **210** in a direction parallel to an imaginary plane formed by a first surface **234** of the cartridge, and the blood exits through the blood outlet **215** in a direct normal to an imaginary plane formed by the first surface **234.**

The housing may define a sweep gas inlet **220** and a sweep gas outlet **225.** The sweep gas inlet **220** and sweep gas outlet **225** may be coupled by one or more hollow fibers **240.** In some embodiments, a single hollow fiber may be used to couple the sweep gas inlet **220** and the sweep gas outlet **225.** In some embodiments, the hollow fiber may be configured to spiral around a central axis **231.** In some embodiments, each blood-gas exchange cartridge may be configured to have a gas exchange surface area of between 1.5 m² and 2.5 m². In some embodiments, each blood-gas exchange cartridge may be configured to have a gas exchange surface area of at between 0.5 m² and 1.5 m².

In some embodiments, the blood may be in contract with one or more hollow fibers. In some embodiments, the hollow fibers allow oxygen molecules from the sweep gas to enter into the blood. In some embodiments, the hollow fibers allow carbon dioxide molecules from the blood to enter the sweep gas.

In some embodiments, each blood gas exchange cartridge may comprise at least one sensor 250. In some embodiments, at least one sensor may be configured to detect when the cartridge has been attached to both the first oxygenator manifold and the second oxygenator manifold. In some embodiments, at least one sensor may be configured to detect the presence of blood in the cartridge. In some embodiments, at least one sensor may be configured to detect the flow of blood in the cartridge.

In some embodiments, each cartridge may include circuitry **260,** which may include one or more processors. In some embodiments, each cartridge may include a visual indicator **265,** indicating a status of the cartridge. For example, in some embodiments, the indicator may be red if the cartridge is not ready for use, yellow if it is on standby, and green if it is in use. The circuitry **260** may be configured to determine the status based on the one or more sensors.

In some embodiments, one or more electrical contacts and/or communication interfaces **270** may be included in each cartridge. In some embodiments, each cartridge may be configured to receive electrical current through an electrical contact when the cartridge is positioned correctly. In some embodiments, each cartridge may be configured to pass information through an electrical contact and/or communication interface **270.** For example, information from a sensor **250** may be passed to a controller through an electrical contact and/or communication interface **270.**

In some embodiments, each of the plurality of blood-gas exchange cartridges is disposable.

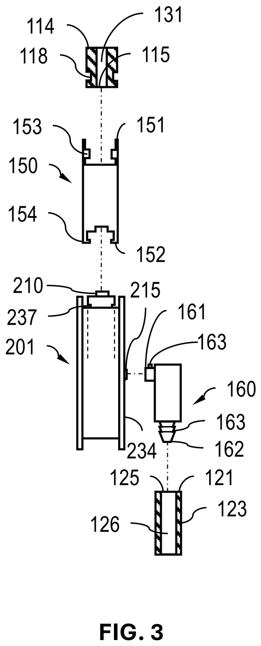

Referring to FIG. 3, in some embodiments, the system may utilize one or more connectors **150, 160.**

In some embodiments, the system may include a first connector **150** operably connected to a blood inlet **210** and a second connector **160** operably connected to a blood outlet **215.**

In some embodiments, the first connector may have a first end **151** and a second end **152.** The first end may be removably coupled to the first oxygenator manifold. For example, the first end may have protrusions or depressions **153** that are configured to interact with the depressions or protrusions **118** on an arm **114** of the first manifold. In some embodiments, the first connector operably couples the blood-gas exchange cartridge with a lumen **131** in the first manifold.

In some embodiments, the second end may be removably coupled to a blood-gas exchange cartridge. For example, the second end may have protrusions or depressions **154** that are configured to interact with the depressions or protrusions **237** on a blood-gas exchange cartridge.

In some embodiments, the second connector may have a first end **161** and a second end **162.** The first end may be removably coupled to a blood gas exchange cartridge. For example, the first end may be configured to interact with the blood outlet **215.** In some embodiments, the second connector may have one or more protrusions or depressions **163** at or near the first end configured to interact with the blood gas exchange cartridge to prevent the second connector from moving in a direction normal to a first surface **234** of the blood-gas exchange cartridge.

In some embodiments, the second end may be removably coupled the second oxygenator manifold. For example, the second end may have protrusions or depressions **163** that are configured to interact with a blood port **125** at a first end **121** of an arm **123** of the second manifold. In some embodiments, the second connector operably couples the blood-gas exchange cartridge with a lumen **126** in the second manifold.

In some embodiments, the first connector and the second connector may both be configured to form at least one fluid tight seal.

In some embodiments, the first and second connectors each may include at least one single use connectors. In some embodiments, the first connector and the second connector may be configured to lock into place when connected.

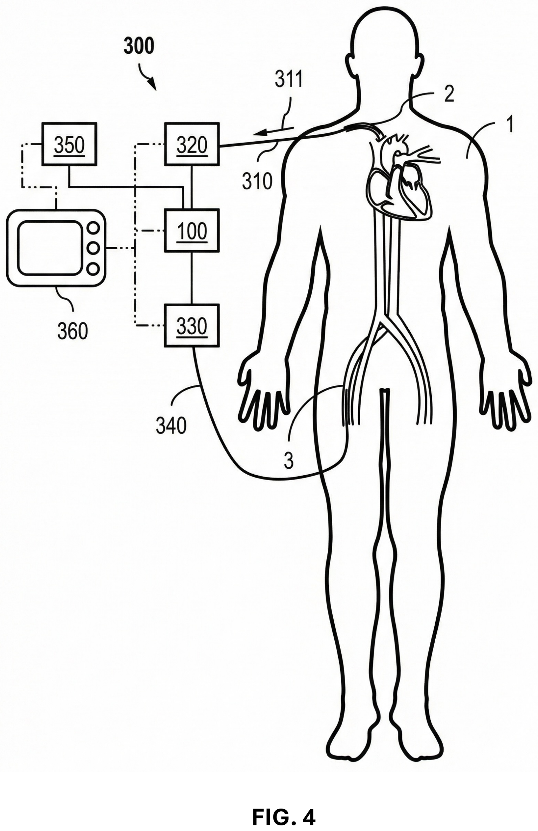

Referring to FIG. 4, a schematic of an expanded system **300** for blood-gas exchange can be seen. The system may include tubing **310** configured to be operably coupled to a first blood vessel **2** of a patient **1.** The tubing **310** may be configured to allow blood to flow in a first direction **311** away from the patient **1.**

The tubing may be operably coupled to a pump **320.** The outlet from the pump may be operably coupled to an inlet to the modular oxygenation device **100.** The pump may be configured to control the flow of blood through the modular oxygenation device **100.**

The system may include a heat exchanger **330** operably coupled to the modular oxygenation device **100.** The heat exchanger may be upstream or downstream from the modular oxygenation device **100.**

A sweep gas source **350** may be operably coupled to the modular oxygenation device **100.**

A second tube **340** may be configured to be operably coupled to a second blood vessel **3** in the patient **1.** The second tube **340** may be operably coupled to the output of the modular oxygenation device **100.**

The system may include a controller **360** configured to interact with the modular oxygenation device **100,** the pump **320,** the heat exchanger **330,** and/or the sweep gas source **350.**

In some embodiments, the controller may be configured to control at least one valve of the system. The controlled valve may be a valve **119** in the first oxygenator manifold, a valve **129** in the second oxygenator manifold, or both.

In some embodiments, the controller may be configured to receive information from one or more sensors from one or more blood-gas exchange cartridges. In some embodiments, the controller may be configured to control at least one valve of the system in response to information received from the at least one sensor.

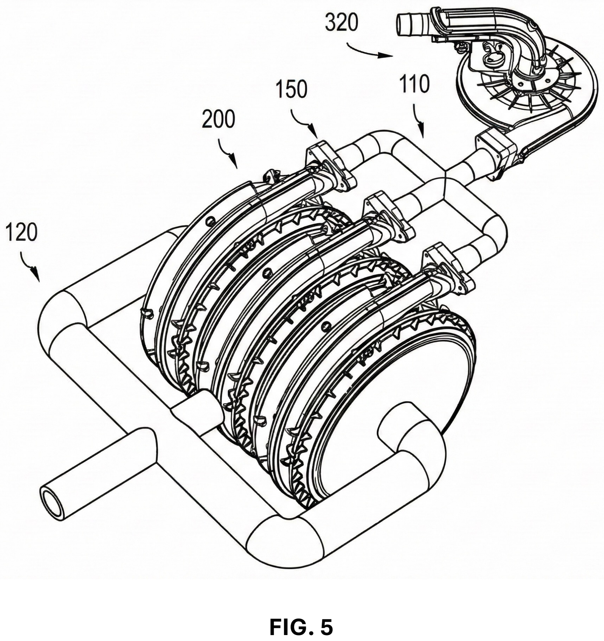

Referring to FIG. 5, in some embodiments, another example of a system can be seen, with three blood-gas exchange cartridges **200** coupled to the first oxygenator manifold **110** and second oxygenator manifold **120.** A pump **320** may be directly coupled to the first oxygenator manifold. A first connector **150** is seen coupling the first oxygenator manifold **110** to each blood-gas exchange cartridge **200.**

In some embodiments, a kit may be provided. The kit may include a first oxygenator manifold as described herein. The kit may include a second oxygenator manifold as described herein. The kit may include a plurality of blood-gas exchange cartridges as described herein. The kit may include a heat exchanger. The kit may include a pump. The kit may include one or more valves. The kit may include a controller. The controller may be configured to control at least one valve of the kit.

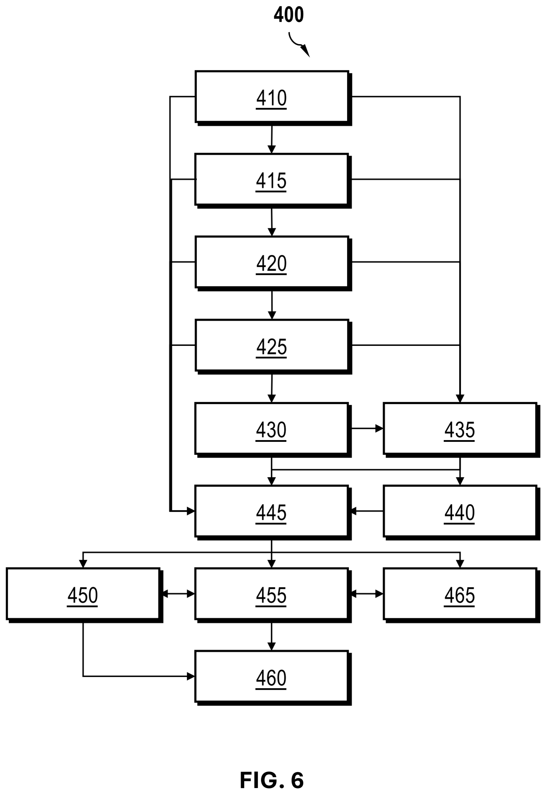

A method for exchanging gas may be provided. Referring to FIG. 6, the method **400** may include flowing **410** a gas through a first blood-gas exchange cartridge of a plurality of blood-gas exchange cartridges.

Each of the plurality of blood gas cartridges may be removable connected to first and second oxygenator manifolds. The first oxygenator manifold may include a plurality of first blood ports, a first blood port removably connected to the first blood-gas exchange cartridge. The second oxygenator manifold may include a plurality of second blood ports, and the first blood-gas exchange cartridge may be removably connected to a second blood port of a plurality of second blood ports.

The method may include flowing **415** blood through the first oxygenator manifold while the gas is flowing through the first blood-gas exchange cartridge.

In some embodiments, the method may include flowing **420** blood through a second blood-gas exchange cartridge of the plurality of blood gas exchange cartridges. In some embodiments, this may be done automatically, by a controller configured to control one or more valves. In some embodiments, this may include flowing blood through both the first and second blood-gas exchange cartridges for at least a period of time. In some embodiments, the period of time may be a predetermined period of time. In some embodiments, the period of time may be based on a predicted residence time of blood in the blood-gas exchange cartridge. For example, if a cartridge can hold 1 L of blood, a pump is configured to pump 5 L of blood per minute through the system, and the valve is being controlled in a way that allows only 20% of the blood to enter the second blood-gas exchange cartridge, the predetermined time may be 1 minute (1 liter / 20% x 5 liters per minute = 1 minute). In some embodiments, the first and second blood-gas exchange cartridges are not utilized simultaneously (*e.g*., blood is only pumped through either the first or the second blood-gas exchange cartridge at any given point in time).

In some embodiments, the method may include stopping **425** blood flow through the first blood gas-exchange cartridge after flowing blood through the second blood-gas exchange cartridge.

In some embodiments, the method may include removing **430** the first blood-gas exchange cartridge from the first and second oxygenator manifolds.

In some embodiments, the method may include connecting **435** a third blood-gas exchange cartridge to the first and second oxygenator manifolds.

In some embodiments, the method may include flowing **440** blood through the third blood gas-exchange cartridge. In some embodiments, blood flow through the third blood-gas exchange cartridge while blood is flowing through the first blood-gas exchange cartridge. In some embodiments, blood flow through the third blood-gas exchange cartridge while blood is flowing through the second blood-gas exchange cartridge.

In some embodiments, the method may include receiving **445** information from a first sensor configured to determine a status of a blood-gas exchange cartridge. In some embodiments, the sensor may be included in a blood-gas exchange cartridge. In some embodiments, a sensor may be included in the first and/or second manifold.

In some embodiments, the method may include determining **450** if a blood-gas exchange cartridge is attached based on the information from the first sensor. For example, in some embodiments, a sensor on a blood-gas exchange cartridge is configured to detect if an electrical current is being applied to an electrical contact. The electrical contact may be configured so it only received power when it is positioned correctly with respect to the first and second manifolds. If the sensor is detecting current, the blood-gas exchange cartridge may be attached.

In some embodiments, the method may include determining **455** if an unused blood-gas exchange cartridge is attached based on the information from the first sensor. For example, in some embodiments, an optical sensor may be configured to detect a wavelength of light that would be absorbed by blood. When the sensor fails to receive the wavelength of light, it indicates blood may be present in the device. If blood is present, the cartridge has been used. Conversely, if the sensor is unused, the sensor should continue to receive the wavelength of light.

In some embodiments, the method may include generating **460** an alarm responsive to a determination **450** a blood-gas exchange cartridge is not attached, a determination **455** an unused blood-gas exchange cartridge is not attached, or both.

In some embodiments, the method may include controlling **465** one or more valves based on the information received from the first sensor.

In some embodiments, a controller may be configured to cause the blood to flow through appropriate blood-gas exchange cartridges, control valves, receive information from the sensors, make determinations regarding whether cartridges and/or unused cartridges are attached, and generating alarms.

Specific embodiments may be understood as described below.

In a first embodiment, a system for blood-gas exchange may be provided. The system may include: (i) a first oxygenator manifold comprising a plurality of first blood ports; (ii) a second oxygenator manifold comprising a plurality of second blood ports; (iii) a plurality of blood-gas exchange cartridges, wherein each of the plurality of blood-gas exchange cartridges is configured to be removably coupled to the first oxygenator manifold and the second oxygenator manifold, each of the plurality of blood-gas exchange cartridges being configured to allow blood and a sweep gas to flow through the removably attachable cartridge and configured to allow a gas molecule to transfer between the sweep gas and the blood.

In a second embodiment, based on the first embodiment, each of the plurality of blood-gas exchange cartridges is configured to be removably coupled to at least one of the plurality of first blood ports and at least one of the plurality of second blood ports.

In a third embodiment, based on the second embodiment, the first oxygenator manifold comprises a blood inlet, each of the plurality of first blood ports being operably connected to the blood inlet.

In a fourth embodiment, based on the third embodiment, the first oxygenator manifold further comprises at least one valve, where each of the plurality of first blood ports is operably connected to the at least one valve.

In a fifth embodiment, based on the fourth embodiment, the at least one valve includes a plurality of valves.

In a sixth embodiment, based on any one of the first through fifth embodiments, the second oxygenator manifold comprises a blood outlet, each of the plurality of first blood ports being operably connected to the blood outlet.

In a seventh embodiment, based on the sixth embodiment, the second oxygenator manifold further comprises at least one valve, where each of the plurality of first blood ports is operably connected to the at least one valve.

In an eighth embodiment, based on the seventh embodiment, the at least one valve includes a plurality of valves.

In a ninth embodiment, based on any one of the first through eighth embodiments, the plurality of blood-gas exchange cartridges includes a first blood-gas exchange cartridge and a second blood-gas exchange cartridge, wherein the first and second blood-gas exchange cartridges are arranged in parallel.

In a tenth embodiment, based on the ninth embodiment, the first oxygenator manifold and the second oxygenator manifold are expandable to allow a third blood-gas exchange cartridge to be added to the system.

In an eleventh embodiment, based on the ninth embodiment, blood is configured to flow through each of the first and second blood-gas exchange cartridge simultaneously.

In a twelfth embodiment, based on the ninth embodiment, blood is configured to flow through one of the first and second blood-gas exchange cartridges while the other of the first and second blood-gas exchange cartridges is configured to remain in standby mode.

In a thirteenth embodiment, based on any one of the first through twelfth embodiments, the system comprises a number C of blood-gas exchange cartridges, and a number V of valves, such that C > V.

In a fourteenth embodiment, based on any one of the first through twelfth embodiments, the system comprises a number C of blood-gas exchange cartridges, and a number V of valves, such that C = V.

In a fifteenth embodiment, based on any one of the first through twelfth embodiments, the system comprises a number C of blood-gas exchange cartridges, and a number V of valves, such that C < V.

In a sixteenth embodiment, based on any one of the first through fifteenth embodiments, each blood-gas exchange cartridge comprises at least one sensor.

In a seventeenth embodiment, based on the sixteenth embodiment, the system further includes a controller configured to control at least one valve of the system.

In an eighteenth embodiment, based on the seventeenth embodiment, the controller is configured to control at least one valve of the system in response to information received from the at least one sensor.

In a nineteenth embodiment, based on any one of the first through eighteenth embodiments, each blood-gas exchange cartridge is configured to have a gas exchange surface area of between 1.5 m² and 2.5 m².

In a twentieth embodiment, based on any one of the first through eighteenth embodiments, each blood-gas exchange cartridge is configured to have a gas exchange surface area of at between 0.5 m² and 1.5 m².

In a twenty-first embodiment, based on any one of the first through twentieth embodiments, the system further includes a first connector operably connected to a blood inlet and a second connector operably connected to a blood outlet.

In a twenty-second embodiment, based on the twenty-first embodiment, (i) the first connector is removably coupled to one of the plurality of blood-gas exchange cartridges, the first oxygenator manifold, or both; (ii) the second connector is removably coupled to one of the plurality of blood-gas exchange cartridges, the second oxygenator manifold, or both; and/or (iii) the first connector and the second connector are configured to form a fluid tight seal.

In a twenty-third embodiment, based on any one of the first through twenty-second embodiments, the first and second connectors each include single use connectors.

In a twenty-fourth embodiment, based on any one of the twenty-first embodiment through the twenty-third embodiment, the first connector and the second connector are each configure to lock into place when connected.

In a twenty-fifth embodiment, based on any one of the first through twenty-fourth embodiments, the first oxygenator manifold is configured to be removably connected to 5 or more blood-gas exchange cartridges.

In a twenty-sixth embodiment, based on any one of the first through the twenty-fifth embodiments, the system is configured to operate simultaneously using at least two, but less than all, of the plurality of blood-gas exchange cartridges.

In a twenty-seventh embodiment, based on any one of the first through the twenty-sixth embodiments, the system further includes tubing operably coupled to the plurality of blood-gas exchange cartridges.

In a twenty-eighth embodiment, based on any one of the first through the twenty-seventh embodiments, the system further includes a heat exchanger operably coupled to the plurality of blood-gas exchange cartridges.

In a twenty-ninth embodiment, based on any one of the first through the twenty-eighth embodiments, the system further includes a pump operably coupled to the plurality of blood-gas exchange cartridges.

In a thirtieth embodiment, based on any one of the first through the twenty-ninth embodiments, each of the plurality of blood-gas exchange cartridges is disposable.

In a thirty-first embodiment, a kit may be provided. The kit includes: (i) a first oxygenator manifold comprising a plurality of first blood ports; (ii) a second oxygenator manifold comprising a plurality of second blood ports; and (iii) a plurality of blood-gas exchange cartridges, each blood-gas exchange cartridge capable of being removably coupled to the first and second oxygenator manifolds, each blood-gas exchange cartridge configured to allow blood and a sweep gas to flow through the removably attachable cartridge and configured to allow a gas molecule to transfer between the sweep gas and the blood.

In a thirty-second embodiment, based on the thirty-first embodiment, each of the plurality of blood-gas exchange cartridges is configured to be removably coupled to at least one of the plurality of first blood ports and at least one of the plurality of second blood ports.

In a thirty-third embodiment, based on the thirty-first or thirty-second embodiment, the kit further includes a heat exchanger.

In a thirty-fourth embodiment, based on any one of the thirty-first through the thirty-third embodiments, the kit may further include a pump.

In a thirty-fifth embodiment, based on any one of the thirty-first through the thirty-fourth embodiments, the first oxygenator manifold is configured to be removably connected to 5 or more blood-gas exchange cartridges.

In a thirty-sixth embodiment, based on any one of the thirty-first through the thirty-fifth embodiments, the first oxygenator manifold comprises a blood inlet and each of the plurality of first blood ports are operably connected to the blood inlet.

In a thirty-seventh embodiment, based on the thirty-sixth embodiment, the first oxygenator manifold further comprises at least one valve, where each of the plurality of first blood ports is connected to the at least one valve.

In a thirty-eighth embodiment, based on the thirty-seventh embodiment, the at least one valve is a plurality of valves.

In a thirty-ninth embodiment, based on any one of the thirty-first through the thirty-eighth embodiments, the second oxygenator manifold comprises a blood outlet and each of the plurality of first blood ports are operably connected to the blood outlet.

In a fortieth embodiment, based on the thirty-ninth embodiment, the second oxygenator manifold further comprises at least one valve, where each of the plurality of first blood ports is connected to the at least one valve.

In a forty-first embodiment, based on the fortieth embodiment, the at least one valve is a plurality of valves.

In a forty-second embodiment, based on any one of the thirty-first through forty-first embodiments, the kit includes a number C of blood-gas exchange cartridges, and a number V of valves, such that C > V.

In a forty-third embodiment, based on any one of the thirty-first through forty-first embodiments, the kit includes a number C of blood-gas exchange cartridges, and a number V of valves, such that C = V.

In a forty-fourth embodiment, based on any one of the thirty-first through forty-first embodiments, the kit includes a number C of blood-gas exchange cartridges, and a number V of valves, such that C < V.

In a forty-fifth embodiment, based on any one of the thirty-first through forty-fourth embodiments, each blood-gas exchange cartridge comprises at least one sensor.

In a forty-sixth embodiment, based on the forty-fifth embodiment, the kit further includes a controller configured to control at least one valve of the kit.

In a forty-seventh embodiment, based on the forty-sixth embodiment, the controller is configured to control at least one valve of the system in response to information received from the at least one sensor.

In a forty-eighth embodiment, based on any one of the thirty-first through forty-seventh embodiments, each blood-gas exchange cartridge is configured to have a gas exchange surface area of between 1.5 m² and 2.5 m².

In a forty-ninth embodiment, based on any one of the thirty-first through forty-seventh embodiments, each blood-gas exchange cartridge is configured to have a gas exchange surface area of at between 0.5 m² and 1.5 m².

In a fiftieth embodiment, based on any one of the thirty-first through forty-ninth embodiments, the kit includes a first connector operably connected to a blood inlet and a second connector operably connected to a blood outlet.

In a fifty-first embodiment, based on the fiftieth embodiment, (i) the first connector is removably coupled to one of the plurality of blood-gas exchange cartridges, the first oxygenator manifold, or both; (ii) the second connector is removably coupled to one of the plurality of blood-gas exchange cartridges, the second oxygenator manifold, or both; and/or (iii) the first connector and the second connector are configured to form a fluid tight seal.

In a fifty-second embodiment, based on the fiftieth or fifty-first embodiment, the first connector and the second connector are each configure to lock into place when connected.

In a fifty-third embodiment, based on the fifty-second embodiment, the first connector, the second connector, or both comprises a single-use connection.

In a fifty-fourth embodiment, based on the fifty-third embodiment, the first connector, the second connector, or both comprises a reusable connection.

In a fifty-fifth embodiment, based on any one of the thirty-first through fifty-fourth embodiments, the kit further includes tubing configured to be operably coupled to the plurality of blood-gas exchange cartridges.

In a fifty-sixth embodiment, a method for exchanging gas may be provided, the method including: (i) flowing a gas through a first blood-gas exchange cartridge of a plurality of blood-gas exchange cartridges, each of the plurality of blood gas cartridges being removable connected to first and second oxygenator manifolds; and (ii) while the gas is flowing through the first blood-gas exchange cartridge, flowing blood through the first oxygenator manifold, the first oxygenator manifold comprising a plurality of first blood ports, at least one first blood port removably connected to the first blood-gas exchange cartridge, the first blood-gas exchange cartridge further removably connected to a blood port of a plurality of second blood ports of the second oxygenator manifold.

In a fifty-seventh embodiment, based on the fifty-sixth embodiment, the method may further include flowing blood through a second blood-gas exchange cartridge of the plurality of blood gas exchange cartridges.

In a fifty-eighth embodiment, based on the fifty-sixth or fifty-seventh embodiment, the method further includes stopping blood flow through the first blood gas-exchange cartridge after flowing blood through the second blood-gas exchange cartridge.

In a fifty-ninth embodiment, based on the fifty-eighth embodiment, the method further includes removing the first blood-gas exchange cartridge from the first and second oxygenator manifolds.

In a sixtieth embodiment, based on the fifty-eighth embodiment, the method further includes connecting a third blood-gas exchange cartridge to the first and second oxygenator manifolds.

In a sixty-first embodiment, based on the sixtieth embodiment, the method further includes flowing blood through the third blood gas-exchange cartridge while blood is flowing through the first blood-gas exchange cartridge.

In a sixty-second embodiment, based on any one of the fifty-sixth through sixty-first embodiments, the method further includes receiving information from a first sensor configured to determine a status of a blood-gas exchange cartridge.

In a sixty-third embodiment, based on the sixty-second embodiment, the method further includes determining if a blood-gas exchange cartridge is attached based on the information from the first sensor.

In a sixty-fourth embodiment, based on the sixty-second or sixty-third embodiment, the method further includes determining if an unused blood-gas exchange cartridge is attached based on the information from the first sensor.\

In a sixty-fifth embodiment, based on the sixty-third or sixty-fourth embodiment, the method further includes generating an alarm responsive to a determination a blood-gas exchange cartridge is not attached, a determination an unused blood-gas exchange cartridge is not attached, or both.

In a sixty-sixth embodiment, based on any one of the sixty-second through sixty-fifth embodiments, the method further includes controlling a valve based on the information received from the first sensor.

Embodiments of the present disclosure are described in detail with reference to the figures wherein like reference numerals identify similar or identical elements. It is to be understood that the disclosed embodiments are merely examples of the disclosure, which may be embodied in various forms. Well known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Those skilled in the art will recognize or be able to ascertain using no more than routine experimentation many equivalents to the specific embodiments of the invention described herein. Such equivalents are intended to be encompassed by the following claims.

## Claims

1. A system for blood-gas exchange, comprising:
a first oxygenator manifold comprising a plurality of first blood ports; and
a second oxygenator manifold comprising a plurality of second blood ports;
a plurality of blood-gas exchange cartridges,
wherein each of the plurality of blood-gas exchange cartridges is configured to be removably coupled to the first oxygenator manifold and the second oxygenator manifold, each of the plurality of blood-gas exchange cartridges being configured to allow blood and a sweep gas to flow through the removably attachable cartridge and configured to allow a gas molecule to transfer between the sweep gas and the blood.

2. The system according to claim 1, wherein each of the plurality of blood-gas exchange cartridges is configured to be removably coupled to at least one of the plurality of first blood ports and at least one of the plurality of second blood ports.

3. The system according to claim 2, wherein the first oxygenator manifold comprises a blood inlet, each of the plurality of first blood ports being operably connected to the blood inlet.

4. The system according to claim 3, wherein the first oxygenator manifold further comprises at least one valve, where each of the plurality of first blood ports is operably connected to the at least one valve.

5. The system according to any one of claims 1-4, wherein the second oxygenator manifold comprises a blood outlet, each of the plurality of first blood ports being operably connected to the blood outlet, and optionally wherein the second oxygenator manifold further comprises at least one valve, where each of the plurality of first blood ports is operably connected to the at least one valve.

6. The system according to any one of claims 1-5, wherein the plurality of blood-gas exchange cartridges includes a first blood-gas exchange cartridge and a second blood-gas exchange cartridge, wherein the first and second blood-gas exchange cartridges are arranged in parallel, and optionally wherein the first oxygenator manifold and the second oxygenator manifold are expandable to allow a third blood-gas exchange cartridge to be added to the system.

7. The system according to any one of claims 1-6, wherein each blood-gas exchange cartridge comprises at least one sensor.

8. The system according to claim 7, further comprising a controller configured to control at least one valve of the system, and optionally wherein the controller is configured to control at least one valve of the system in response to information received from the at least one sensor.

9. A kit comprising:
a first oxygenator manifold comprising a plurality of first blood ports;
a second oxygenator manifold comprising a plurality of second blood ports; and
a plurality of blood-gas exchange cartridges, each blood-gas exchange cartridge capable of being removably coupled to the first and second oxygenator manifolds, each blood-gas exchange cartridge configured to allow blood and a sweep gas to flow through the removably attachable cartridge and configured to allow a gas molecule to transfer between the sweep gas and the blood.

10. The kit according to claim 9, wherein each of the plurality of blood-gas exchange cartridges is configured to be removably coupled to at least one of the plurality of first blood ports and at least one of the plurality of second blood ports.

11. A method for exchanging gas, comprising:
flowing a gas through a first blood-gas exchange cartridge of a plurality of blood-gas exchange cartridges, each of the plurality of blood gas cartridges being removable connected to first and second oxygenator manifolds; and
while the gas is flowing through the first blood-gas exchange cartridge, flowing blood through the first oxygenator manifold, the first oxygenator manifold comprising a plurality of first blood ports, at least one first blood port removably connected to the first blood-gas exchange cartridge, the first blood-gas exchange cartridge further removably connected to a blood port of a plurality of second blood ports of the second oxygenator manifold.

12. The method according to claim 11,
further comprising flowing blood through a second blood-gas exchange cartridge of the plurality of blood gas exchange cartridges; and/or
further comprising stopping blood flow through the first blood gas-exchange cartridge after flowing blood through the second blood-gas exchange cartridge.

13. The method according to claim 12,
further comprising removing the first blood-gas exchange cartridge from the first and second oxygenator manifolds; or
further comprising connecting a third blood-gas exchange cartridge to the first and second oxygenator manifolds.

14. The method according to claim 13, further comprising flowing blood through the third blood gas-exchange cartridge while blood is flowing through the first blood-gas exchange cartridge.

15. The method according to any one of claims 11-14, further comprising receiving information from a first sensor configured to determine a status of a blood-gas exchange cartridge.
